(19) Europäisches Patentamt
European Patent Office
Office européen des brevets

(11) Publication number: 0 120 108
B1

# (12) EUROPEAN PATENT SPECIFICATION

(45) Date of publication of patent specification: 13.08.86

(51) Int. Cl.$^4$: G 01 N 27/30, G 01 N 27/48, G 01 N 27/46 // A61B5/00

(21) Application number: 83102976.4

(22) Date of filing: 25.03.83

## (54) Electrochemical cell for determining a particular component of a fluid.

(43) Date of publication of application:
03.10.84 Bulletin 84/40

(45) Publication of the grant of the patent:
13.08.86 Bulletin 86/33

(84) Designated Contracting States:
CH DE FR GB IT LI NL SE

(58) References cited:
FR-A-2 441 166
GB-A-1 505 343

MESURES-REGULATION-AUTOMATISME, vol. 46, no. 5, May 1981, Paris, "Des techniques microélectroniques pour un capteur d'oxygène dissous",page 85

MEDICAL & BIOLOGICAL ENGINEERING & COMPUTING, vol. 21, no. 1, January 1981, Stevenage, G.S.MARGULES et al. "Hydrogel bases in vivo reference electrode catheter", pages 1-8

MEDICAL & BIOLOGICAL ENGINEERING & COMPUTING, vol. 16, no. 5, September 1978, Stevenage, D. PARKER et al. "Catheter-tip electrode for continuous measurement of pO2 and pCO2", pages 599-600

(73) Proprietor: HONEYWELL MEDICAL ELECTRONICS B.V.
I.B.C.-Weg 1
NL-5683 PK Best (NL)

(72) Inventor: Kuypers, Martinus Henricus
Schoolstraat 53
NL-5561 AH Riethoven (NL)
Inventor: Vervoort, Martinus Petrus Pancratius
Oranje Nassaulaan 99
NL-5491 HL Sint Oedenrode (NL)

(74) Representative: Rentzsch, Heinz et al
Honeywell Europe S.A. Holding KG Patent- und Lizenzabteilung Kaiserleistrasse 55
D-6050 Offenbach am Main (DE)

(58) References cited:
MEDICAL & BIOLOGICAL ENGINEERING & COMPUTING, vol. 18, no. 6, February 1980, Stevenage, K. SHIMADA et al." Application of catheter-tip i.s.f.e.t. for continuousin vivo measurement", pages 741-745

EP 0 120 108 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to an electrochemical cell in accordance with the general portion of claim 1, and known from Mesures-Regulation-Automatisme, vol. 46, No. 5, May 1981, Paris "Des techniques microélectroniques pour un capteur d'oxygène dissous", page 85. Cells of this type are for instance used for determining the oxygen content of a fluid such as blood or air. A well-known type of this cell is the so-called Clark Cell as described in US—A—29 13 386. For medical and biological applications a miniaturized cell of this type has been proposed in EP—A—103031 (published 21.03.84) wherein a thin layer of hydrophylic polymer is provided between a non-porous membrane and the electrodes and covers at least the cathode. The membrane has at least one hole in it for allowing an electrolytic component of the fluid to enter said hydrophylic polymer layer, whereat the electrolyte is supplied by the fluid under measurement. It is further known from Medical & Biological Engineering & Computing, Vol. 18, No. 6, February 1980, Stevenage K. Shimada et al. "Application of catheter-tip i.s.f.e.t. for continuous in vivo measurement", pages 741—745, to use a hydrogel polymer to improve the blood combatibility, the response time and the reproducibility of a catheter-tip pH sensor.

It is the main object of the present invention to provide an electrochemical cell of the above-mentioned type having a very short response time such that it can be used for the measurement of quickly changing components in a fluid such as the oxygen content or oxygen-partial pressure during respiration.

Also this new cell should be capable of being miniaturized and should be suitable for medical and biological applications, i.e. sterilization should be no problem. These objects and further improvements are accomplished by the invention as characterized in claim 1. Preferred embodiments and improvements are described in the sub-claims.

The invention will be described in more detail with reference to the accompanying drawings in which

Fig. 1 shows an electrochemical cell for measuring the oxygen-partial pressure in a gaseous fluid, e.g. in the respiration air;

Fig. 2 shows at an enlarged scale a top view on the sensor chip;

Fig. 3 is a sectional view of the cell provided in a catheter; and

Fig. 4 is a cross sectional view taken along line IV—IV of Fig. 3.

In Fig. 1 a tube-like housing 1 made of metal, glass, plastic or preferably made of ceramics is supported by an adaptor base plate 2, by means of which the sensor can be attached to a thermostatically controlled part 3 of the mouthpiece of a respiration system. A heating coil 27 surrounds these sensor portions of housing 1 when inserted into part 3. A temperature controller (not shown) accomplished a uniform temperature of the sensor portion during measurement. Housing 1 forms an electrolyte compartment 4 which is closed at the rearward end by means of a plate 5 having a filling opening 6, which after filling is closed by a plug 7 or otherwise. A supporting structure 8 of semicircular cross section is provided in the front end opening of housing 1 and supports a semiconductor substrate 9 forming the active portion of the sensor. The remaining area of the front end surface of housing 1 is closed by a plate 10.

Substrate 9 carries a cathode layer 20 deposited on substrate 9 by the well-known technology of printed circuits, thin film or thick film procedures. The cathode portion 20 and part of the substrate 9 are covered by a layer 12 consisting of a hydrophylic matrix polymer or hydrogel. A selectively permable membrane 13 covers layer 12, whereat inside housing 1 a portion 14 of layer 12 is not covered by the membrane and is directly exposed to the electrolyte in compartment 4. Substrate 9 may also carry an integrated temperature sensor 15 (see Fig. 2) and if required, a heater (not shown) which in a similar manner like the cathode layer can be deposited by thin film or thick film technology. Preferably the heater 27 is located in part 3. Electrical connectors 16 are provided on the substrate for connecting the cathode, the anode, the temperature sensor and the heater to an external measuring and supply circuit by means of a cable 17 and connector pins 18. The contact area 16 is covered and electrically insulated by a protective layer 19 consisting of silicone rubber or any other suitable material. The cathode 20 might be provided as a thin layer between two anode layers 11 on substrate 9 (see Fig. 2). The anode can also be formed by a separate anode 11a projecting into the electrolyte compartment 4 (see Fig. 1). The hydrophylic matrix polymer layer 12 by its capillary action forms a salt bridge between the electrolyte compartment 4 and the cathode layer 20.

Membrane 13 which limits the diffusion of gas to the cathode area of substrate 9 can be produced by a combination of centrifugal spinning technique and lithography which is well-known in the field of thin film technology. By this technology extremely thin membranes can be made in the order of less than 1 μ which leads to a very fast response of the sensor. Other depositing techniques suitable for producing the membrane are spraying, flow coating, silk screening, condensation out of the gas phase, grafting and sputtering. Electrolyte compartment 4 contains a sufficient amount of electrolyte for achieving a long operation time of the sensor. The temperature sensor 15 allows compensation of the temperature dependency of the sensor by means of the electrical monitoring and measuring circuitry connected to terminals 18. If a separate anode 11a is used instead of an anode 11 provided on the substrate 9 itself, the hydrophylic polymer layer 12 forms a salt bridge for the electrolyte. Substrate 9 preferably made of a

semiconducting material such as silicon may also include an integrated amplifier to overcome problems which during long term operation may be caused by parasitic insulation currents or electrostatic effects.

The sensor can be stored dry, and the electrolyte filled in shortly before the sensor will be used. The electrolyte within compartment 4 then by means of the capillary action of hydrophylic polymer layer 12 is brought to the cathode (and anode) area on substrate 9, therewith activating the sensor. If the sensor is used as a polarographic oxygen sensor, the oxygen surrounding the outwardly projecting portion of substrate 9 will diffuse through membrane 13 and the hydrophylic matrix polymer layer 12 to the cathode and will react at the cathode as follows:

$$O_2+4H_2O+4e \rightarrow 4OH^1$$

The corresponding reaction at the anode can be described by

$$4CL'+4Ag \rightarrow 4AgCL+4e$$

in case a silver anode and a chloride containing electrolyte are used. Both the chloride and the hydroxyl ions will be transported from and to the electrolyte compartment 4 via the hydrophylic matrix polymer layer 12. The use of separate anode 11a provides a longer lifetime of the sensor compared with an anode 11 provided on the substrate 9 because the amount of anode material is an essential factor for the timelife. The sensor has a small response time, in particular less than one second, and therewith can be used for monitoring quickly changing oxygen content in a gas stream.

The new sensor can also be used for measuring the content of oxygen or another component of a liquid. Figures 3 and 4 show an embodiment in which the sensor is located in the tip portion of a catheter 1. The supporting structure 8 preferably made of glass or another suitable material carries substrate 9 together with cathode layer 20, hydrophylic matrix polymer layer 12 and membrane 13. The front end portion of closed tube 1 is filled with epoxy resin or silicone rubber 21. A hole 22 provided at the circumferential wall of tube 1 allows the liquid to engage membrane 13 so that oxygen ions can diffuse through membrane 13 and polymer layer 12 to anode 11. An intermediate wall 23 closes electrolyte compartment 4 against the sensor portion of the catheter. The anode might be provided on the substrates 9 or as shown in Figure 3 may be a separate anode 11a held in a supporting tube 24 within catheter 1. Cable 17 and supporting tube 24 are sealed against tube 1 by epoxy resin seals 25, therewith closing electrolyte compartment 4 at the other end. Similar sealings are provided at 26 in the area where cable 17 is connected to substrate 9. An opening provided in seal 25 allows filling compartment 4 with an electrolyte and is thereafter closed by a plug 7.

This type of catheter sensor can also be used for ion-selective and enzymatic sensor constructions. In these cases membrane 13 contains special organic molecules and forms a so-called liquid ion membrane. Such liquid ion membrane materials can also be deposited on top of the hydrogel layer 12 by means of lithographic technology. Liquid ion membranes constitute an effective means for sensing different types of organic components of biological liquids. The use of such liquid ion membranes is described in an article "Ion-Selective Electrodes in Clinical Chemistry" published in *Medical Progress Through Technology* 5.1—12 (1977), pages 1—10, in particular page 4. Since this kind of application involves measuring the difference of electrical potential across a high impedance membrane, environmental electrical disturbance signals may influence the measuring signal which further could be decreased by insulation leakage. To overcome these problems an impendance conversion of the measuring signal is helpful. For this purpose the silver/silver chloride electrode can be connected to the input of an impedance converter, e.g. to the gate electrode if a field effect transistor, therewith converting the high impedance measuring signal to a low impedance output signal. The silver/silver chloride reference electrode can be situated outside the membrane at various positions, e.g. on the substrate, in the lumen of a catheter which is continuously flushed with saline or in the bag or bottle containing the saline.

The invention leads to a versatile sensor construction, which may be used for sensing various types of components of gaseous or liquid fluids, has a fast response, can easily be miniaturized and produced in large quantities by the well-known technology of integrated circuits, lithography and thin film methods.

It is not restricted to medical applications but can also be used in the field of industrial process monitoring and control, bio-technology, environmental protection and for other purposes. Other components of a gaseous or liquid medium (fluid) than those as described above may be monitored or searched-for by the sensor.

**Claims**

1. Electrochemical cell for determining the content or partial pressure of components in a fluid comprising

a) a cathode (20) provided on an insulating substrate (9) and spaced therefrom an anode (11, 11a);

b) a space (4) communicating with said electrodes and adapted for being filled with an electrolyte thus defining a closed electrolyte compartment;

c) a selectively permeable membrane (13) separating said electrodes from the fluid characterized in that

d) a thin layer (12) of hydrophylic polymer is provided between the membrane (13) and the

cathode (20) and covers the cathode and part of the substrate (9);

e) a part of the substrate together with a part of the layer covered by the membrane is adapted to be exposed to the fluid;

f) another part (14) of the layer (12) is in direct contact with the closed electrolyte compartment (4) to serve as a duct for replenishment of electrolyte at the electrodes (11, 11a, 20).

2. Cell according to claim 1, characterized in that the anode (11) is provided on the substrate (9).

3. Cell according to claim 1, characterized in that the anode (11a) is provided separately in the electrolyte compartment (4).

4. Cell according to one of claims 1 to 3, characterized in that the cathode (20), the hydrogel layer (12) and the membrane (13) are made by thin film technology on the substrate (9).

5. Cell according to claim 4, characterized in that the membrane (13) is made by centrifugal spinning technique or other depositing techniques and lithography.

6. Cell according to one of claims 1 to 5, characterized in that the substrate (9) is held in a tube (1) by means of a supporting structure (8) and is provided with electrical connectors (16) for at least the cathode (20).

7. Cell according to one of claims 1 to 6, characterized by a temperature sensor (15) provided on the substrate (9).

8. Cell according to one of claims 1 to 7, characterized in that the substrate (9) is a semiconductor substrate comprising an integrated impedance converter and amplifier or a current amplifier.

9. Cell according to one of claims 1 to 8 for measuring in a gaseous fluid, characterized in that the electrolyte compartment (4) is formed by a tube-like housing (1) and a part of the substrate (9) together with a part of the layer (12) covered by the membrane (13) projects outside from one end wall (10) of the housing.

10. Cell according to one of claims 1 to 8, characterized in that the housing (1) forms part of a thermostatically controlled adapter (3) for becoming inserted into an opening of patient mouthpiece.

11. Cell according to one of claims 1 to 8 for invasive measurement by means of a catheter, characterized in that an opening (22) is provided in the circumferential wall in the tip of the catheter (1), said opening allowing access of the fluid to the membrane (13).

12. Cell according to claim 11, characterized in that the membrane (13) is a liquid ion membrane.

13. Cell according to claim 12, characterized in that the liquid ion membrane is deposited on the top of the hydrogel layer (12) by thin film technology.

**Patentansprüche**

1. Elektrochemische Zelle zur Bestimmung des Gehalts oder Partialdrucks eines Bestandteils in einem Fluid mit

a) einer auf einem isolierenden Substrat (9) angeordneten Kathode (20) sowie einer im Abstand hiervon vorgesehenen Anode (11, 11a);

b) einem mit den genannten Elektroden in Verbindung stehenden, mit einem Elektrolyten zu füllenden Raum (4), der eine Elektrolytkammer bildet;

c) einer die Elektroden vom Fluid trennenden selektiv durchlässigen Membran (13);

dadurch gekennzeichnet, daß

d) eine dünne Schicht (12) eines hydrophilen Polymers zwischen der Membran (13) und der Kathode (20) vorgesehen ist und die Kathode sowie einen Teil des Substrats (9) bedeckt;

e) ein Teil des Substrats zusammen mit einem Teil der von der Membran bedeckten Schicht dem Fluid aussetzbar ist;

f) ein anderer Teil (14) der Schicht (12) in direktem Kontakt mit der Elektrolytkammer (4) steht und als Zuleitung für die Zufuhr des Elektrolyten zu den Elektroden (11, 11a, 20) dient.

2. Zelle nach Anspruch 1, dadurch gekennzeichnet, daß sich die Anode (11) auf dem Substrat (9) befindet.

3. Zelle nach Anspruch 1, dadurch gekennzeichnet, daß die Anode (11a) getrennt in der Elektrolytkammer (4) angeordnet ist.

4. Zelle nach einem der Ansprüche 1 bis 3, dadurch gekennzeichnet, daß die Kathode (20), die Hydrogelschicht (12) und die Membran (13) mittels Dünnschichttechnik auf dem Substrat angebracht sind.

5. Zelle nach Anspruch 4, dadurch gekennzeichnet, daß die Membran (13) durch ein Schleuderverfahren oder eine andere Ablagerungstechnik sowie Lithographie hergestellt ist.

6. Zelle nach einem der Ansprüche 1 bis 5, dadurch gekennzeichnet, daß das Substrat (9) mit Hilfe eines Trägers (8) in einem Rohr (1) gehalten und mit elektrischen Anschlüssen (16) für zumindest die Kathode (20) versehen ist.

7. Zelle nach einem der Ansprüche 1 bis 6, dadurch gekennzeichnet, daß auf dem Substrat (9) ein Temperaturfühler (15) angeordnet ist.

8. Zelle nach einem der Ansprüche 1 bis 7, dadurch gekennzeichnet, daß das Substrat ein Halbleitersubstrat mit einem integrierten Impedanzwandler und Verstärker oder einem Stromverstärker ist.

9. Zelle nach einem der Ansprüche 1 bis 8 Zum Messen in einem gasförmigen Fluid, dadurch gekennzeichnet, daß die Elektrolytkammer (4) durch ein rohrförmiges Gehäuse (1) gebildet ist und ein Teil des Substrats (9) zusammen mit einem von der Membran (13) bedeckten Teil der Schicht (12) aus der einen Endwand (10) des Gehäuses herausragt.

10. Zelle nach einem der Ansprüche 1 bis 8, dadurch gekennzeichnet, daß das Gehäuse (1) Teil eines thermostatisch geregelten Adapters (3) ist, der in eine Öffnung eines Patientenmundstücks einsetzbar ist.

11. Zelle nach einem der Ansprüche 1 bis 8 zur

invasiven Messung mittels Katheter, dadurch gekennzeichnet, daß eine Öffnung (22) in der Umfangswand der Katheterspitze vorgesehen ist und den Zutritt des Fluids zur Membran (13) ermöglicht.

12. Zelle nach Anspruch 11, dadurch gekennzeichnet, daß die Membran (13) eine ionenselektive Flüssigmembran ist.

13. Zelle nach Anspruch 12, dadurch gekennzeichnet, daß die ionenselektive Flüssigmembran durch Dünnfilmtechnik auf der Hydrogelschicht (12) aufgebracht ist.

**Revendications**

1. Cellule électrochimique pour la détermination de la teneur ou de la pression partielle de constituants dans un fluide comprenant

a) une cathode (20) ménagée sur un substrat isolant (9) et, à une certaine distance, une anode (11, 11a);

b) un espace (4) communiquant avec les dites électrodes et adapté pour être rempli d'un électrolyte en définissant ainsi un compartiment d'électrolyte fermé;

c) une membrane sélectivement perméable (13) séparant les dites électrodes du fluide, caractérisée en ce que

d) une couche mince (12) d'un polymère hydrophile est ménagée entre la membrane (13) et la cathode (20) et recouvre la cathode et une partie du substrat (9);

e) une partie du substrat ainsi qu'une partie de la couche recouverte par la membrane est adaptée pour être exposée au fluide;

f) une autre partie (14) de la couche (12) est en contact direct avec le compartiment d'électrolyte fermé (4) pour servir de conduite de remplissage de l'électrolyte au niveau des électrodes (11, 11a, 20).

2. Cellule selon la revendication 1, caractérisée en ce que l'anode (11) est ménagée sur le substrat (9).

3. Cellule selon la revendication 1, caractérisée en ce que l'anode (11a) est ménagée séparément dans le compartiment de l'électrolyte (4).

4. Cellule selon l'une quelconque des revendications 1 à 3, caractérisée en ce que la cathode (20), la couche d'hydrogel (12) et la membrane (13) sont fabriquées sur le substrat (9) par une technologie de film mince.

5. Cellule selon la revendication 4, caractérisée en ce que la membrane (13) est fabriquée par une technique de filage par centrifugation ou d'autres techniques de déposition et par lithographie.

6. Cellule selon l'une quelconque des revendications 1 à 5, caractérisée en ce que le substrat (9) est maintenu dans un tube (1) au moyen d'une structure de support (8) et est muni de contacts électriques (16) au moins pour la cathode (20).

7. Cellule selon l'une quelconque des revendications 1 à 6, caractérisée en ce qu'un détecteur de température (15) est ménagé sur le substrat (9).

8. Cellule selon l'une quelconque des revendications 1 à 7, caractérisée en ce que le substrat (9) est un substrat semiconducteur comprenant un transformateur d'adaptation et amplificateur d'impédance intégré ou un amplificateur de courant.

9. Cellule selon l'une quelconque des revendications 1 à 8 pour des mesures dans un fluide gazeux, caractérisée en ce que le compartiment de l'électrolyte (4) est formé par un logement en forme de tube (1) et en ce qu'une partie du substrat (9) ainsi qu'une partie de la couche (12) recouverte par la membrane (13) dépasse par une paroi extrême (10) du logement.

10. Cellule selon l'une quelconque des revendications 1 à 8, caractérisée en ce que le logement (1) fait partie d'un adaptateur thermostaté (3) destiné à s'insérer dans une ouverture d'un embout pour un patient.

11. Cellule selon l'une quelconque des revendications 1 à 8 pour la mesure d'une invasion au moyen d'un cathéter, caractérisée en ce qu'une ouverture (22) est ménagée dans la paroi circulaire du bout du cathéter (1), la dite ouverture permettant au fluide d'accéder à la membrane (13).

12. Cellule selon la revendication 11, caractérisée en ce que la membrane (13) est une membrane ionique liquide.

13. Cellule selon la revendication 12, caractérisée en ce que la membrane ionique liquide est déposée sur le sommet de la couche d'hydrogel (12) par une technologie de film mince.

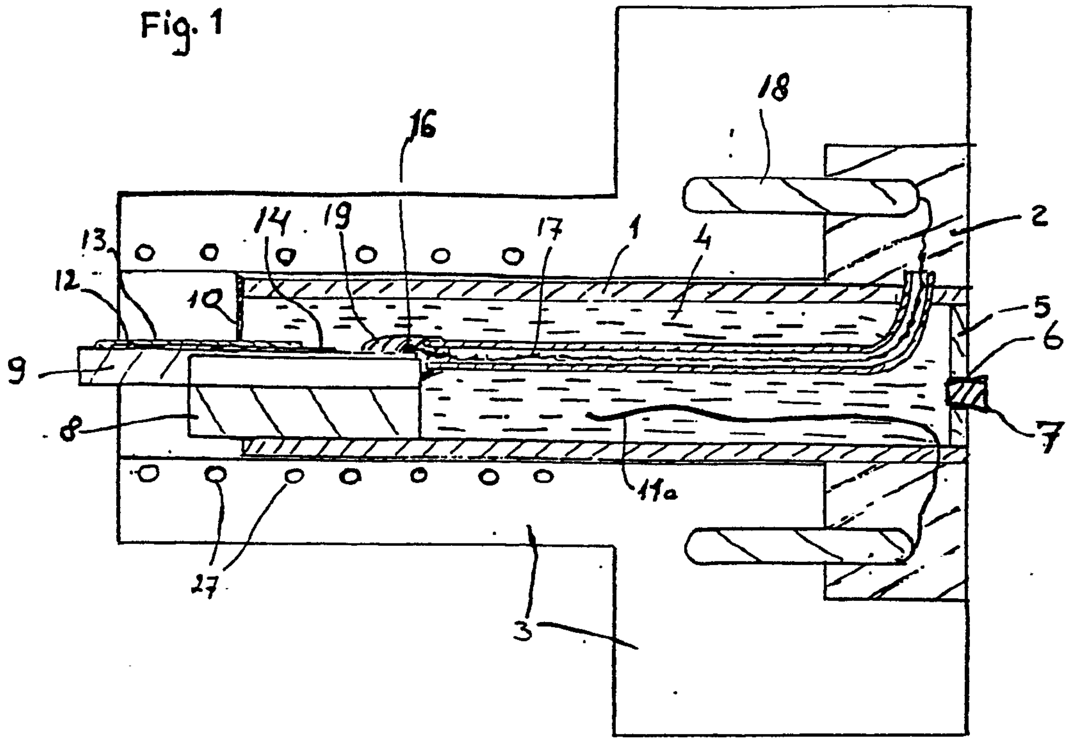


Fig. 1

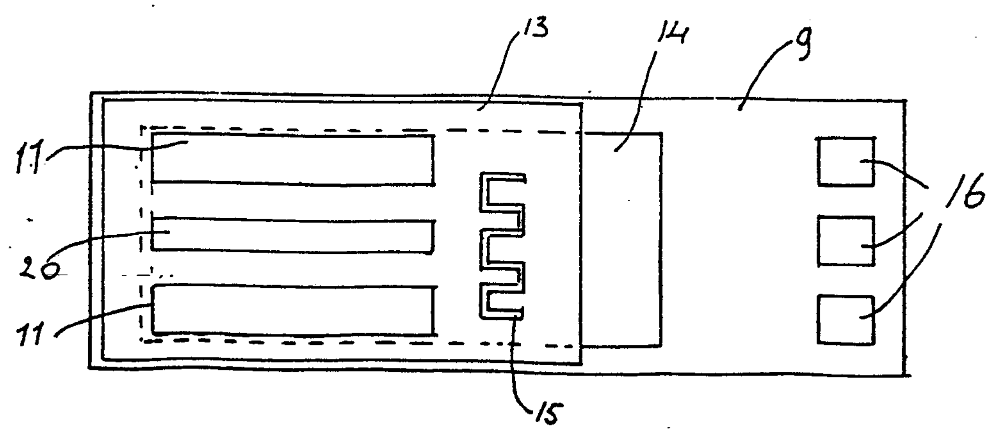


Fig. 2

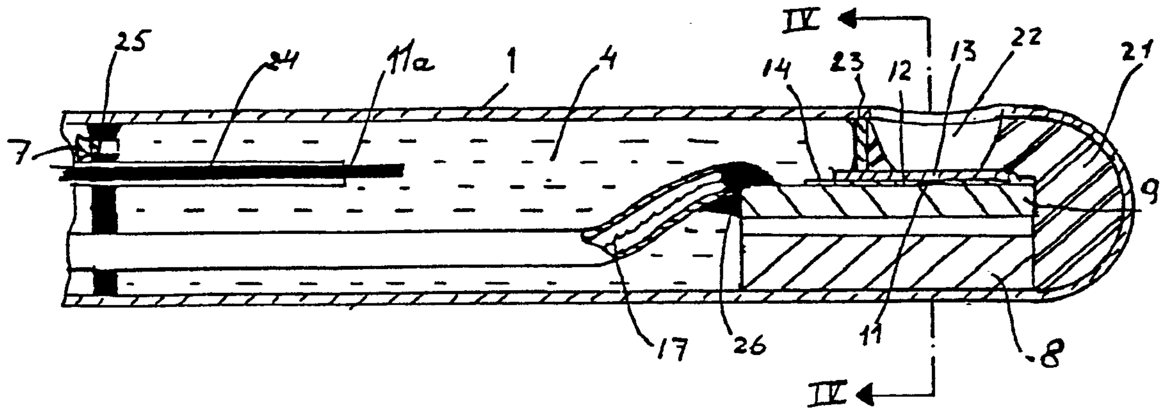


Fig 3

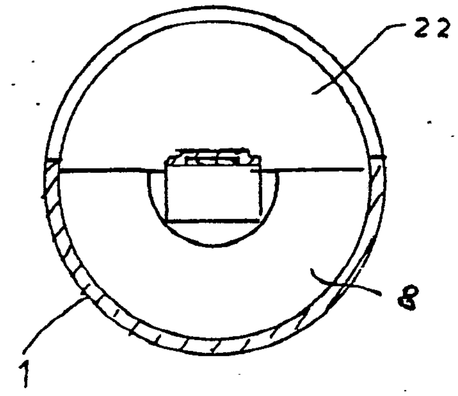


Fig 4